# EUROPEAN PATENT SPECIFICATION

(11) **EP 1 795 891 B1**
(45) Date of publication and mention of the grant of the patent: **18.04.2012**
(21) Application number: 05787571.8
(22) Date of filing: 29.09.2005
(51) Int. Cl.: G01N 31/22, G01N 33/52

(54) **A METHOD FOR PRODUCING MULTILAYER ANALYTICAL ELEMENT**
VERFAHREN ZUR HERSTELLUNG EINES MEHRSCHICHTIGEN ANALYSEELEMENTS
PROCÉDÉ DE FABRICATION D'ÉLÉMENT ANALYTIQUE À COUCHES MULTIPLES

(30) Priority: 30.09.2004 JP 2004286970
(43) Date of publication of application: 13.06.2007
(73) Proprietor: FUJIFILM Corporation, Minato-ku Tokyo 106-0031 (JP)
(72) Inventor: ABE, Yoshihiko, c/o FUJIFILM CORPORATION, Asaka-shi, Saitama 351-8585 (JP)
(74) Representative: Albrecht, Thomas
(86) International application number: PCT/JP2005/017955
(87) International publication number: WO 2006/035875

(56) References cited:
- EP-A1- 0 254 202
- EP-A1- 0 524 596
- JP-A- 8 220 089
- JP-A- 05 026 875
- JP-A- 05 273 207
- JP-A- 10 084 991
- JP-B2- 2 618 727
- JP-B2- 07 013 635
- JP-B2- 07 026 959

## Description

### TECHNICAL FIELD

The present invention relates to a method for producing a dry multilayer analytical element used for clinical diagnoses, food inspection, environmental analysis and the like.

### BACKGROUND ART

In the fields of clinical diagnoses, food inspection, and environmental examination, there is a growing demand for processing a specimen quickly and easily, and dry analytical elements are generally employed to meet such needs. In a dry analytical element, the developing layer, which is used for the reception, development and diffusion of blood or the like, has been typically formed of a fibrous porous material, as described in JP Patent Publication (Kokai) Nos. 55-164356 A (1980), 57-66359 A (1982), and 60-222769 A (1985), for example.

The fibrous porous material has a high spreading rate upon spotting of a liquid sample and is easy to handle during manufacture. It is also compatible with viscous samples, such as whole blood, and is therefore widely used.

In the relevant fields, increasingly higher measurement accuracies (reproducibility) are being required, and several inconveniences have been identified in the fibrous porous material (fabric developing layer). One of the inconveniencies relates to the problem of lot variations in the fabric. Normally, the fabric developing layer is available in woven material and knitted material, and lot-to-lot and intra-lot differences in the manner of weaving or knitting have been found. Specifically, the variations involve the number of stitches per unit area, the weight per unit area, and thickness, for example. There are also lot-to-lot and intra-lot differences in the hydrophilicity of the fabric depending on the degree of washing in the material-washing step in an intermediate process. Furthermore, as the fabric developing layer is not smooth, the developing layer must inevitably be wedged into the lower layer if a sufficient adhesive force is to be ensured by the laminating method during manufacturing. As a result, the lower layer is disturbed and is not suitable for analysis requiring high accuracy. The fabric also tends to extend when bonded to the lower layer for structural reasons, often resulting in a change in its gap volume. The change in the gap volume often leads to a change in the area of spreading of a liquid sample upon spotting, thus resulting in the intra-lot difference and preventing an accurate analysis. While there is a growing demand for analysis with smaller sample amounts, the fabric developing layer tends to have increasing variations in the amount of light it reflects as the amount of sample solution is reduced, due to the influence of its stitches. Furthermore, there is the problem that accurate analysis is prevented by the uneven disturbances introduced in the lower layer upon adhesion of the developing layer.

As a technique to replace the fabric developing layer, a method has been proposed whereby a porous film is produced by coating. A typical example is the so-called brush polymer layer (JP Patent Publication (Kokai) No. 49-53888 A (1974)) that takes advantage of the polymer phase transition reaction during coating/drying. Another example is a bead developing layer (JP Patent Publication (Kokai) No. 55-90859 A (1980)) that is formed by coating microbeads. These methods, however, have the disadvantage that the developing layer is weak and tends to become peeled when a sheet-like coated material is rendered into a slide (during processing).

In order to overcome the aforementioned problems, a method has been proposed whereby a pre-formed, homogeneous non-fibrous porous film having a high film strength is laminated as a developing layer (JP Patent Publication (Kokai) No. 49-53888 A (1974); and JP Patent Publication (Kokai) No. 56-97872 A (1981)). Typical methods for laminating such non-fibrous porous film are disclosed in JP Patent Publication (Kokai) No.60-222770 A (1985), JP Patent Publication (Kokai) No.63-219397A (1988), JP Patent Publication (Kokai) No.63-112999A (1988), JP Patent Publication (Kokai) No.62-182652A (1987), and JP Patent Publication (Kokai) No. 7-26959 A (1995). These methods involve wetting the lower layer uniformly with water so as to cause a water-soluble polymer agent in the lower layer to seep up for bonding. Thereafter, another water-soluble polymer is overcoated, resulting in a re-dissolution and an uneven seep-up property, thereby adversely affecting the within-run reproducibility of measurement values.

### DISCLOSURE OF THE INVENTION

### PROBLEMS TO BE SOLVED BY THE INVENTION

It is an object of the invention to solve the aforementioned problems of the background art. Specifically, it is an object of the invention to provide a method for producing a dry multilayer analytical element in which the within-run reproducibility of measurement values is improved.

### MEANS FOR SOLVING THE PROBLEMS

The present inventors have made an intensive research and analysis to solve the aforementioned objects, and have found that the aforementioned objects can be solved by introducing an adhesion layer on an upper surface of at least one functional layer disposed on a support, laminating a non-fibrous porous film on the adhesion layer, and further overcoating a water-soluble polymer on an upper surface of the porous liquid-sample-developing layer. Thus, the present invention has been completed.

Specifically, the invention provides a method for producing a dry multilayer analytical element for the analysis of a liquid sample which comprises providing at least one functional layer on one surface of a water-non-transmitting planar support; providing an adhesion layer on an upper surface of the functional layer; providing at least one porous liquid-sample-developing layer comprising non-fibrous porous film on an upper surface of the adhesion layer; and coating a water-soluble polymer on an upper surface of the porous liquid-sample-developing layer.

Preferably, a non-fibrous porous film is laminated on an upper surface of the adhesion layer while an adhesion layer is being provided by coating a water-soluble polymer solution on an upper surface of the functional layer, thereby providing the porous liquid-sample-developing layer.

Preferably, an adhesion layer is provided by coating an adhesive agent on an upper surface of the functional layer, and then a non-fibrous porous film is laminated on an upper surface of the adhesion layer, thereby providing the porous liquid-sample-developing layer.

Preferably, the non-fibrous porous film comprises: 6, 6-nylon; 6-nylon; acrylate copolymer; polyacrylate; polyacrylonitrile; polyacrylonitrile copolymer; polyamide, polyimide; polyamide-imide; polyurethane; polyether sulfone; polysulfone; a mixture of polyether sulfone and polysulfone; cellulose acylate; a saponified substance of cellulose acylate; polyester; polyester carbonate; polyethylene; polyethylene chlorotrifluoroethylene copolymer; polyethylene tetrafluoroethylene copolymer; polyvinyl chloride; polyolefin; polycarbonate; polytetrafluoroethylene; polyvinylidene difluoride; polyphenylene sulfide; polyphenylene oxide; polyfluorocarbonate; polypropylene; polybenzoimidazole; polymethyl methacrylate; styrene-acrylonitrile copolymer; styrene-butadiene copolymer; a saponified substance of ethylene-vinyl acetate copolymer; polyvinyl alcohol; and a mixture thereof. More preferably, the non-fibrous porous film comprises polysulfone, polyether sulfone, cellulose acylate, 6,6-nylon, or 6-nylon. Preferably, the non-fibrous porous film is an asymmetric film.

In another aspect, the invention provides a dry multilayer analytical element for the analysis of a liquid sample, which is produced by the above production method of the invention and which comprises a water-non-transmitting planar support on one side of which at least one functional layer, an adhesion layer, at least one porous liquid-sample-developing layer comprising non-fibrous porous film, and a water-soluble polymer layer are integrally layered in the mentioned order.

### BEST MODE FOR CARRYING OUT THE INVENTION

Embodiments of the invention are described in the following.

The method for producing a dry multilayer analytical element for the analysis of a liquid sample according to the invention is characterized in that at least one functional layer is provided on one surface of a water-non-transmitting planar support, an adhesion layer is provided on an upper surface of the functional layer, at least one porous liquid-sample-developing layer comprising non-fibrous porous film is provided on an upper surface of the adhesion layer, and then a water-soluble polymer is coated on an upper surface of the porous liquid-sample-developing layer.

The adhesion layer can be provided on the upper surface of the functional layer, and then at least one porous liquid-sample-developing layer comprising non-fibrous porous film can be provided on the upper surface of the adhesion layer by either of the following methods: (1) A method whereby a non-fibrous porous film is laminated on an upper surface of the adhesion layer while the adhesion layer is being provided by coating a water-soluble polymer solution on an upper surface of the functional layer, thereby providing a porous liquid-sample-developing layer; or (2) A method whereby an adhesion agent is coated on an upper surface of the functional layer and dried to provide the adhesion layer, and then a non-fibrous porous film is laminated on an upper surface of the adhesion layer to provide a porous liquid-sample-developing layer.

The water-soluble polymer used in the invention is not particularly limited. Examples are cellulose ethers such as carboxymethylcellulose and methylcellulose; alginic acid and alginic acid derivatives; polyvinyl alcohol and its derivatives; polyacrylic acid and its derivatives; polyethylene glycol; polyethylene oxide; and water-soluble polysaccharide and its derivatives. The polymer may be a copolymer of these or a mixture thereof.

The amount of the polymer used in the form of the adhesion layer is preferably 0.05 to 30 g/m² and more preferably on the order of 0.1 to 10 g/m². While the adhesion force can be increased by increasing the amount of the polymer, the less is better because an increase affects performance in terms of a decrease in the reaction rate of the analytical element, for example.

The adhesive agent used in the invention is not particularly limited as long as it is capable of closely adhering the functional layer and the porous liquid-sample-developing layer comprising a non-fibrous porous film. Example are: cellulose ether; alginic acid; polyvinyl alcohol; polyacrylic acid and its derivatives; polyethylene glycol; polyethylene oxide; water-soluble polysaccharide and its derivatives; polyvinylalkyl ether (polyvinymethyl ether, polyvinyl ethyl ether, polyvinylisobutyl ether, and the like); natural rubber; chloroprene; styrene-butadiene rubber; polymer obtained by copolymerization of acrylate of aliphatic alcohol having carbon number 2 to 16 as a main constituent with a monomer having a polar group such as acrylic acid, allyl acid and the like; silicone adhesive obtained by a combination of silicon rubber and silicon resin; an adhesive comprising a styrene-isoprene-styrene block polymer as a main constituent; rosin resin; terpene resin; hydrogenated hydrocarbon resin; polyisobutylene; indene; dasima; kovar; Picopale; alkyd resin; cellulosic ester; and neoprene. Among those mentioned above, polyvinylalkyl ether is preferable. The adhesive agent may be a hot-melt adhesive, which is a type of adhesive that is solid at room temperature but becomes molten when heated for use. As a hot-melt material, materials described on pages 4-5 of a publication "Kogyo Zairyo (Engineering Materials)," Vol. 26, No. 11 may be used. Specific examples are: ethylene copolymers such as ethylene-vinyl acetate copolymer, ethylene-vinyl acetate copolymer, ethylene ethylacrylate copolymer, and ethylene-acrylate copolymer; polyolefins such as low-molecular-weight polyethylene and atactic polypropylene; polyamides such as nylon; thermoplastic rubber such as polyester copolymer, and styrene block copolymers such as SBS; styrene-butadiene rubber; butyl rubber; urethane rubber; rosin; petroleum resin; terpene resin; paraffin; and synthetic wax.

As the water-non-transmitting planar support, a conventional water-non-transmitting support used in conventional dry analytical elements can be used. For example, it may be a film- or sheet-like support made of a polymer, such as polyethylene terephthalate, bisphenol A polycarbonate, polystyrene, cellulosic ester (such as cellulose diacetate, cellulose triacetate, and cellulose acetate propionate, for example), with a thickness ranging from about 50 µm to about 1 mm, and preferably from about 80 µm to about 300 µm.

An undercoat layer may be provided on the surface of the support as needed, whereby the adhesion between the adhesion layer and the support can be made stronger. Instead of such undercoat layer, the surface of the support may be physically or chemically activated so as to enhance its adhesion force.

The dry multilayer analytical element of the invention comprises a porous liquid-sample-developing layer comprising at least one non-fibrous porous film. The porous liquid-sample-developing layer is a layer with the function of spreading a component in an aqueous specimen in a planar fashion without substantially causing the component to be unevenly distributed, so that the component can be supplied to the functional layer at a substantially constant ratio per unit area.

The number of porous liquid-sample-developing layers is not limited to one; it may comprise a laminate of two or more layers of non-fibrous porous films bonded by an adhesive that is partially located. The porous liquid-sample-developing layer may also include a spread-control agent, such as a hydrophilic polymer, in order to control its spreading property. Further, a reagent for causing a desired detection reaction, a reagent for promoting the detection reaction, a variety of reagents for reducing or preventing an interfering or blocking reaction, or some of these reagents may be contained.

The porous liquid-sample-developing layer of the invention comprises a non-fibrous porous film. Preferably, the non-fibrous porous film is a porous film made of an organic polymer, which film may be either symmetric or asymmetric. In the case of an asymmetric porous film, the asymmetry ratio is preferably 2.0 or more. In the case of a symmetric porous film, the asymmetry ratio is preferably not more than 2.0. The asymmetric porous film herein refers to a porous film having a larger mean diameter of pores on one surface than that on the other surface. The asymmetry ratio refers to the value obtained by dividing the larger mean pore diameter with the smaller mean pore diameter.

Preferable examples of the porous film made of an organic polymer include: 6, 6-nylon; 6-nylon; acrylate copolymer; polyacrylate; polyacrylonitrile; polyacrylonitrile copolymer; polyamide, polyimide; polyamide-imide; polyurethane; polyether sulfone; polysulfone; a mixture of polyether sulfone and polysulfone; cellulose acylate; a saponified substance of cellulose acylate; polyester; polyester carbonate; polyethylene; polyethylene chlorotrifluoroethylene copolymer; polyethylene tetrafluoroethylene copolymer; polyvinyl chloride; polyolefin; polycarbonate; polytetrafluoroethylene; polyvinylidene difluoride; polyphenylene sulfide; polyphenylene oxide; polyfluorocarbonate; polypropylene; polybenzoimidazole; polymethyl methacrylate; styrene-acrylonitrile copolymer; styrene-butadiene copolymer; a saponified substance of ethylene-vinyl acetate copolymer; polyvinyl alcohol; and a mixture thereof.

Of these, more preferable are: 6, 6-nylon; 6-nylon; polyether sulfone; polysulfone; a mixture of polyether sulfone and polysulfone; cellulose acylate; a saponified substance of cellulose acylate; polyester; polyethylene; polypropylene; polyolefin; polyacrylonitrile; polyvinyl alcohol; polycarbonate; polyester carbonate; polyphenylene oxide; polyamide; polyimide; polyamide-imide; and a mixture thereof.

More preferable examples are polysulfone, polyether sulfone, cellulose acylate; 6,6-nylon, and 6-nylon; particularly more preferable examples are polysulfone and polyether sulfone; a most preferable example is polysulfone.

The thickness of the non-fibrous porous film is preferably 80 to 300 µm; more preferably it is 100 to 200 µm; particularly preferably it is 130 to 160 µm.

The mean pore diameter of the non-fibrous porous film is preferably 0.3 to 10 µm; more preferably it is 0.45 to 5 µm.

In one example (1) of the dry multilayer analytical element for liquid sample analysis according to the invention, one or a plurality of functional layers are disposed on the transparent support, and further a porous liquid-sample-developing layer is disposed on the functional layer. In another example (2), one or a plurality of functional layers are disposed on the transparent support, and further, on the functional layer, there is disposed a porous liquid-sample-developing layer that contains a reagent for sample analysis. Thus, the porous liquid-sample-developing layer of the invention may or may not contain a reagent for sample analysis.

In the case of the porous liquid-sample-developing layer containing a reagent, a porous film may be immersed in a reagent solution and then dried so as to produce a reagent-containing film. In another method, the porous film may be coated with a reagent solution, which is then dried so as to produce a reagent-containing non-fibrous porous film; the method, however, is not particularly limited.

In the method for producing a dry multilayer analytical element according to the present invention, a water-soluble polymer is coated on an upper surface of the non-fibrous porous film which is used as the porous liquid-sample-developing layer. By coating a water-soluble polymer, the water-soluble polymer is contained in the porous liquid-sample-developing layer in such a manner that the water-soluble polymer does not interact with the functional layer.

The polymer which is coated on the porous liquid-sample-developing layer is not limited as long as it is a water-soluble polymer. Examples include: cellulose ethers such as carboxymethylcellulose, methylcellulose, and hydroxypropylcellulose; alginic acid and alginic acid derivatives; polyvinyl alcohol and its derivatives; polyacrylic acid and its derivatives; polyethylene glycol; polyethylene oxide; and water-soluble polysaccharide its derivatives. The polymer may be a copolymer of the mentioned examples or a mixture thereof.

The amount of the water-soluble polymer dispersed in the developing layer is preferably 0.1 to 10 g/m²; more preferably it is 1.0 to 5 g/m².

The dry multilayer analytical element of the invention includes at least one functional layer. The number of the functional layers is not particularly limited; it may be one or two or more, for example.

Examples of the functional layer include: a water-absorbing layer for absorbing a liquid reagent; a mordant layer for preventing the diffusion of a dye produced by chemical reaction; a gas transmitting layer for selectively transmitting gas; an intermediate layer for suppressing or promoting the transport of substance between layers; an elimination layer for eliminating an endogenous substance; a light-shielding layer for enabling a stable reflective photometry; a color shielding layer for suppressing the influence of an endogenous dye; a separation layer for separating blood cells and plasma; a reagent layer containing a reagent that reacts with a target of analysis; and a coloring layer containing a coloring agent.

In an example of the invention, a hydrophilic polymer layer may be provided on the support as a functional layer via another layer as needed, such as an underlayer. The hydrophilic polymer layer may include: a non-porous, water-absorbing and water-permeable layer basically consisting only of a hydrophilic polymer; a reagent layer comprising a hydrophilic polymer as a binder and including some or all of a coloring agent that is directly involved in a coloring reaction; and a detection layer containing a component (such as a dye mordant) that immobilizes the coloring agent in the hydrophilic polymer.

In the following, the functional layers are described.

### (Reagent layer)

The reagent layer is a water-absorbing and water-permeable layer comprising a hydrophilic polymer binder in which at least some of a reagent composition that reacts with a detected component in an aqueous liquid to produce an optically detectable change is substantially uniformly dispersed. The reagent layer includes an indicator layer and a coloring layer.

A hydrophilic polymer that can be used as the binder in the reagent layer is generally a natural or synthetic hydrophilic polymer with a swelling rate ranging from about 150% to about 2000%, and preferably from about 250% to about 1500%, at 30°C, upon water absorption. Examples of such a hydrophilic polymer include: gelatin (such as acid-treated gelatin or deionized gelatin, for example) disclosed in JP Patent Publication (Kokai) No. 60-108753 A (1985); a gelatin derivative (such as phthalated gelatin or hydroxyacrylate graft gelatin, for example); agarose; pullulan; pullulan derivative; polyacrylamide; polyvinyl alcohol; and polyvinylpyrrolidone.

The reagent layer may be a layer appropriately cross-linked and cured using a crosslinking agent. Examples of the crosslinking agent include: for gelatin, known vinylsulfon crosslinking agent, such as 1, 2-bis(vinylsulfonyl acetoamide)ethane and bis(vinylsulfonylmethyl)ether, and aldehydes; and, for methallyl alcohol copolymer, aldehydes and epoxy compounds containing two glycidyl groups and the like.

The thickness of the reagent layer when dried is preferably in the range of about 1 µm to about 100 µm, and more preferably about 3 µm to about 30 µm. Preferably, the reagent layer is substantially transparent.

The reagent contained in the reagent layer or other layers in the dry multilayer analytical element of the invention may be appropriately selected depending on the tested substance to be detected.

For example, when analyzing ammonia (in cases where the tested substance is ammonia or ammonia-producing substance), examples of a coloring ammonia indicator include: leuco dyes, such as leucocyanine dye, nitro-substituted leuco dye, and leucophthalein dye (see U.S. Patent No. Re. 30267 or JP Patent Publication (Kokoku) No. 58-19062 B (1983); pH indicators, such as bromophenol blue, bromocresol green, bromthymol blue, quinoline blue, and rosolic acid (see Encyclopaedia Chimica, Vol. 10, pp 63-65, published by Kyoritsu Shuppan K. K.); triarylmethane dye precursors; leucobenzylidene dyes (see JP Patent Publication (Kokai) Nos. 55-379 A (1980) and 56-145273 A (1981)); diazonium salt and azo dye couplers; and base bleaching dyes. The content of the coloring ammonia indicator with respect to the weight of the binder is preferably in the range of about 1 to about 20% by weight.

The reagent that reacts with an ammonia-producing substance as a tested substance to produce ammonia is preferably an enzyme or a reagent that contains an enzyme; the enzyme suitable for analysis may be selected appropriately depending on the type of the ammonia-producing substance as the tested substance. When an enzyme is used as the regent, the combination of the ammonia-producing substance and the reagent is determined by the specificity of the enzyme. Examples of the combination of the ammonia-producing substance and an enzyme as the reagent include: urea/urease; creatinine/creatinine deiminase; amino acid/amino-acid dehydrogenase; amino acid/amino-acid oxidase; amino acid/ammonia lyase; amine/amine oxidase; diamine/amine oxidase; glucose and phosphoamidate/phosphoamidate-hexose phosphotransferase; ADP/carbamate kinase and carbamoyl phosphate; acid amide/amide hydrolase; nucleobase/nucleobase deaminase; nucleoside/nucleoside deaminase; and nucleotide/nucleotide deaminase; guanine/guanase. An alkaline buffer that can be used in the reagent layer during the analysis of ammonia may be a buffer with a pH of 7.0 to 12.0, and preferably 7.5 to 11.5.

In addition to the reagent that reacts with an ammonia-producing substance to produce ammonia, an alkaline buffer, and a hydrophilic polymer binder with a film-forming capability, the reagent layer for the analysis of ammonia may include a wetting agent, a binder crosslinking agent (curing agent), a stabilizing agent, a heavy-metal ion trapping agent (complexing agent), and the like, as needed. The heavy-metal ion trapping agent is used for masking heavy-metal ions that hinder enzyme activity. Examples of the heavy-metal ion trapping agent include complexanes such as: EDTA·2Na; EDTA·4Na; nitrilotriacetic acid (NTA); and diethylenetriaminepentaacetic acid.

Examples of the glucoses-measuring reagent composition include glucose oxidase, peroxidase, 4-aminoantipyrine or derivatives thereof, and an improved Trinder's reagent composition including 1,7-dihydroxynaphthalene, as described in U.S. Patent No. 3,992,158, JP Patent Publication (Kokai) Nos. 54-26793 A (1979), 59-20853 A (1984), 59-46854 A (1984), and 59-54962 A (1984).

### (Light-shielding layer)

A light-shielding layer may be provided on top of the reagent layer as needed. The light-shielding layer is a water-transmitting or water-permeable layer comprising a small amount of hydrophilic polymer binder with a film-forming capability in which particles with light-absorbing or light-reflecting property (together referred to as "light-shielding property") are dispersed. The light-shielding layer blocks the color of the aqueous liquid supplied to the developing layer (to be described later) by spotting, particularly the color red of hemoglobin in the case where the sample is whole blood, when measuring detectable changes (in color or in coloration, for example) that developed in the reagent layer by reflection photometry from the light-transmitting support side. In addition, the light-shielding layer also functions as a light-reflecting layer or a background layer.

Examples of the particle with light-reflecting property include: titanium dioxide particles (microcrystalline particles of rutile type, anatase type, or brookite type, with a particle diameter of about 0.1 µm to about 1.2 µm); barium sulfate particles; aluminum particles; and microflakes. Examples of the light-absorbing particles include: carbon black, gas black, and carbon microbeads, of which titanium dioxide particles and barium sulfate particles are preferable. Particularly, anatase-type titanium dioxide particles are preferable.

Examples of the hydrophilic polymer binder with a film-forming ability include regenerated cellulose of weak hydrophilicity and cellulose acetate, in addition to hydrophilic polymers similar to the hydrophilic polymer used for the manufacture of the aforementioned reagent layer. Of these, gelatin, gelatin derivatives, and polyacrylamide are preferable. Gelatin or gelatin derivatives may be used in a mixture with a known curing agent (crosslinking agent).

The light-shielding layer may be provided by applying an aqueous dispersion of light-shielding particles and a hydrophilic polymer onto the reagent layer by a known method and then drying. Alternatively, instead of providing the light-shielding layer, a light-shielding particle may be contained in the aforementioned developing layer.

### (Water-absorbing layer)

The dry multilayer analytical element of the invention may be provided with a water-absorbing layer between the support and the reagent layer. The water-absorbing layer is a layer consisting primarily of a hydrophilic polymer that becomes swollen by absorbing water, so that it can absorb water in the aqueous liquid sample that has reached or permeated the boundary of the water-absorbing layer. The water-absorbing layer functions to promote the permeation of blood plasma, which is the aqueous liquid component in the case where the sample is whole blood, to the reagent layer. The hydrophilic polymer used in the water-absorbing layer may be selected from those used in the aforementioned reagent layer. For the water-absorbing layer, gelatin, gelatin derivatives, polyacrylamide, and polyvinyl alcohol are generally preferable. Particularly, the aforementioned gelatin and deionized gelatin are preferable. Most particularly, the aforementioned gelatin used in the reagent layer is preferable. The thickness of the water-absorbing layer when dried is about 3 µm to about 100 µm, preferably about 5 µm to about 30 µm. The amount of coating is about 3 g/m² to about 100 g/m², and preferably about 5 g/m² to about 30 g/m². The pH of the water-absorbing layer upon use (during the implementation of analysis operation) may be adjusted by adding a pH buffer or a known basic polymer or the like in the water-absorbing layer, as will be described later. The water-absorbing layer may further contain a known dye mordant or a polymer dye mordant, for example.

### (Detection layer)

The detection layer is generally a layer in which a dye or the like produced in the presence of a detected component is diffused and becomes optically detectable through a light-transmitting support. The detection layer may consist of a hydrophilic polymer, and it may contain a dye mordant, such as a cationic polymer for an anionic dye, for example. The water-absorbing layer generally refers to a layer in which the dye produced in the presence of the detected component is not substantially diffused, and it is distinguished from the detection layer in this respect.

The reagent layer, water-absorbing layer, developing layer and the like may each contain a surface active agent, of which one example is a nonionic surface active agent. Examples of nonionic surface active agent include: p-octylphenoxypolyethoxyethanol, p-nonylphenoxypolyethoxyethanol, polyoxyethylene oleyl ether, polyoxyethylene sorbitan monolaurate, p-nonylphenoxypolyglycidol, and octyl glucoside. By having the nonionic surface active agent contained in the developing layer, its function of spreading the aqueous liquid sample (metering function) can be improved. By having the nonionic surface active agent contained in the reagent layer or the water-absorbing layer, the water in the aqueous liquid sample can be facilitated to be substantially uniformly absorbed by the reagent layer or the water-absorbing layer during analysis operation, so that the contact of the liquid with the developing layer can take place quickly and substantially uniformly.

The tested substance that can be analyzed by the dry multilayer analytical element of the invention is not particularly limited and a particular component in any liquid sample (including bodily fluids, such as whole blood, blood plasma, blood serum, lymph fluid, urine, saliva, cerebrospinal fluid, and vaginal fluid; drinking water, liquors, river water, and factory waste water) can be analyzed. For example, the dry multilayer analytical element can be used for the analysis of albumin (ALB), glucose, urea, bilirubin, cholesterol, proteins, enzymes (including blood enzymes such as lactic dehydrogenase, CPK (creatine kinase), ALT (alanineamino-transferase), AST (aspartate aminotransferase), and GGT (γ-glutamyltranspeptidase)).

The dry multilayer analytical element of the invention can be prepared by known methods. Hemolysis reagent may be added in the reagent solution in advance for application or impregnation. In another method, the developing layer may be coated with an aqueous solution, an organic solvent (ethanol or methanol, for example), or a solution of a water-organic solvent mixture, either alone or containing a surface active agent or a hydrophilic polymer for spread area control, so as to impregnate the developing layer with the hemolysis reagent. The tested substance may be analyzed using this method in accordance with a known method.

For example, the dry multilayer analytical element of the invention may be cut into small pieces of squares with each side measuring about 5 mm to about 30 mm, or circles of similar sizes. They can then be accommodated in a slide frame such as described in JP Patent Publication (Kokoku) No. 57-283331 B (1982) (corresponding to U.S. Patent No. 4169751), JP Utility Model Publication (Kokai) No. 56-142454 U (1981) (corresponding to U.S. Patent No. 4387990), JP Patent Publication (Kokai) No. 57-63452 A (1982), JP Utility Model Publication (Kokai) No. 58-32350 U (1983), and JP Patent Publication (Kohyo) No. 58-501144 A (1983) (corresponding to WO083/00391), and the slide can then be used as a chemical analysis slide. This is preferable from the viewpoint of manufacture, packaging, shipping, storage, measurement operation, and so on. Depending on the purpose of use, the element may be stored in a cassette or a magazine in the form of an elongated tape. Alternatively, such small pieces may be stored in a container with an opening, they may be affixed to or accommodated in an opening card, or the cut pieces may be used as is.

In the dry multilayer analytical element of the invention, about 2 µL to about 30 µL, and preferably 4 µL to 15 µL of an aqueous liquid sample is spotted on the porous liquid-sample-developing layer. The thus spotted dry multilayer analytical element is then incubated at a certain temperature ranging from about 20°C to about 45°C, preferably from about 30°C to about 40°C, for 1 to 10 minutes. The coloration or change in color in the dry multilayer analytical element is measured from the light-transmitting support side by reflection photometry, and the amount of the tested substance in the specimen can be determined using a prepared analytical curve based on the principle of colorimetry.

A highly accurate quantitative analysis can be performed by a very simple procedure using a chemical analyzer such as those disclosed in JP Patent Publication (Kokai) Nos. 60-125543 A (1985), 60-220862 A (1985), 61-294367 A (1986), 58-161867 A (1983) (corresponding to U.S. Patent No. 4,424,191), for example. Depending on the purpose or the desired level of accuracy, the degree of coloration may be judges visually and a semi-quantitative analysis may be performed.

Since the dry multilayer analytical element of the invention is stored in a dry state until the beginning of analysis, there is no need to prepare a reagent as required. Further, as the reagents are generally more stable in a dry state, the dry multilayer analytical element of the invention can be more simply and quickly utilized than the so-called wet methods, in which solutions of reagents must be prepared as required. The invention is also superior as an examination method whereby a highly accurate examination can be performed with small quantities of liquid sample.

The invention will be hereafter described in more detail by way of examples thereof. The invention is not limited by these examples.

### EXAMPLES

### Example 1 (Production of a dry analytical element for the measurement of uric acid having the water-soluble polymer adhesion layer)

A 180-µm colorless, transparent smooth film of polyethylene terephthalate undercoated with gelatin was coated with an aqueous solution (pH=7.0) of the following composition and dried to a thickness of 14 µm.

| | |
|---|---|
| Surface active agent | 11.63 g/m² |
| Gelatin | 16.34 g/m² |
| Boric acid | 0.03 g/m² |
| Potassium chloride | 0.03 g/m² |
| Leuco dye | 0.31 g/m² |
| Uricase | 0.59 KU/m² |
| Peroxidase | 15.09 KU/m² |

Then, a polysulfone film (HS2000 manufactured by Fuji Photo Film Co., Ltd.) was laminated while a polymer aqueous solution of the following composition was being coated as an adhesion layer.

| | |
|---|---|
| Surface active agent | 0.17 g/m² |
| Polyvinyl alcohol | 0.75 g/m² |

On the above porous film, an aqueous solution (pH=9.5) of the following composition was coated and dried.

| | |
|---|---|
| Hydroxypropylcellulose | 3.9 g/m² |
| Boric acid | 0.46 g/m² |
| Potassium chloride | 0.40 g/m² |
| Surface active agent | 0.62 g/m² |

As the surface active agent, polyoxy(2-hydroxy)propylene nonylphenyl ether (Olin surfactant 10G) was used.

The above integral multilayer analytical element was cut into a square chip measuring 12 mm×13 mm, which was then placed in a slide frame (as described in JP Patent Publication (Kokai) No.57-63452 A (1982)), thereby producing a dry analytical element for the analysis of uric acid.

### Example 2 (Production of a dry analytical element for the measurement of uric acid having an adhesive coating)

The lower layer was identical to that of the Example. Instead of coating a water-soluble polymer, an ethanol solution of the following composition was coated and dried, and, immediately before winding, a polysulfone film (HS2000 manufactured by Fuji Photo Film Co., Ltd.) was affixed.

| | |
|---|---|
| Polyvinymethyl ether | 2.6 g/m² |

On the above porous film, an aqueous solution (pH=9.5) of the following composition was coated and dried.

| | |
|---|---|
| Hydroxypropylcellulose | 3.9 g/m² |
| Boric acid | 0.46 g/m² |
| Potassium chloride | 0.40 g/m² |
| Surface active agent | 0.62 g/m² |

With the above integral multilayer analytical element, a dry analytical element for the analysis of uric acid was produced in the same way as in Example 1.

### Comparative Example 1 (Production of a dry analytical element for the measurement of uric acid)

The lower layer was identical to that of Example 1. When laminating a porous film, water was supplied to the entire surface at the volume of approximately 30 g/m², thereby wetting the same. Thereafter, a polysulfone porous film HS200 (manufactured by Fuji Photo Film Co., Ltd.) was laminated.

On the above porous film, an aqueous solution (pH=9.5) of the following composition was coated and dried in the same way as in Example 1.

| | |
|---|---|
| Hydroxypropylcellulose | 3.9 g/m² |
| Boric acid | 0.46 g/m² |
| Potassium chloride | 0.40 g/m² |
| Surface active agent | 0.62 g/m² |

The above integral multilayer analytical element was cut into a square chip measuring 12 mm×13 mm, thereby producing a dry analytical element for the analysis of uric acid in the same way as in Example 1.

### Comparative Example 1 (Production of a dry analytical element for the measurement of uric acid)

The lower layer was identical to that of Example 1. Before laminating the porous film, the following aqueous solution (pH=9.5) was coated and dried such that the following composition was obtained.

| | |
|---|---|
| Hydroxypropylcellulose | 3.9 g/m² |
| Boric acid | 0.46 g/m² |
| Potassium chloride | 0.40 g/m² |
| Surface active agent | 0.62 g/m² |

The layer of the above composition was coated and dried, followed by the laminating of the porous film. Specifically, a polysulfone film (HS2000 manufactured by Fuji Photo Film Co., Ltd.) was laminated while a polymer aqueous solution (with the same composition as in Example 1) of the following composition was being coated as an adhesion layer.

| | |
|---|---|
| Surface active agent | 0.17 g/m² |
| Polyvinyl alcohol | 0.75 g/m² |

With the above integral multilayer analytical element, a dry analytical element for the analysis of uric acid was produced in the same way as in Example 1.

### Comparative Example 3

The lower layer was identical to that of Example 1. Before laminating the porous film, the following aqueous solution (pH=9.5) was coated and dried such that the following composition was obtained.

| | |
|---|---|
| Hydroxypropylcellulose | 3.9 g/m² |
| Boric acid | 0.46 g/m² |
| Potassium chloride | 0.40 g/m² |
| Surface active agent | 0.62 g/m² |

After the layer of the above composition was coated and dried, a porous film was laminated. Specifically, a polymer ethanol solution (having the same composition as in Example 2) of the following composition was coated as an adhesion layer and then dried, and immediately before winding, a polysulfone film (HS2000 manufactured by Fuji Photo Film Co., Ltd.) was laminated.

| | |
|---|---|
| Polyvinymethyl ether | 2.6 g/m² |

With the above integral multilayer analytical element, a dry analytical element for the analysis of uric acid was produced in the same way as in Example 1.

### Test Example (Regarding within-run reproducibility)

The dry analytical elements produced by the methods of Examples 1 and 2 and Comparative Examples 1 to 3 were measured in terms of within-run reproducibility.

Measurement was conducted by spotting 10 µL of a specimen, which comprised human pool serum, on the analytical elements ten times, and using FDC5000 manufactured by Fuji Photo Film Co., Ltd. Measurement values were obtained by converting the reflective OD four minutes after spotting based on calibration curves that were stored in advance. Table 1 shows the CV values upon spotting of the specimen with the density UA=5.5 mg/dL for N=10.

**Table 1**

| | Example 1 | Example 2 | Comparative Example 1 | Comparative Example 2 | Comparative Example 3 |
|---|---|---|---|---|---|
| Example | 2.1% | 1.8% | 3.8% | 7.5% | 4.3% |

The results in Table 1 show that good within-run reproducibility is obtained in cases where the adhesion layer is introduced and further the polymer was dispersed on the adhesion layer as an overcoat.

### INDUSTRIAL APPLICABILITY

In the dry multilayer analytical element of the present invention, an adhesion layer is introduced on an upper surface of the functional layer on a support having at least one functional layer, and a non-fibrous porous film is laminated on the adhesion layer, and further a water-soluble polymer is overcoated on an upper surface of the porous liquid-sample-developing layer. Thus, a dry multilayer analytical element having an improved within-run reproducibility can be provided.

## Claims

1. A method for producing a dry multilayer analytical element for the analysis of a liquid sample which comprises providing at least one functional layer on one surface of a water-non-transmitting planar support; providing an adhesion layer on an upper surface of the functional layer; providing at least one porous liquid-sample-developing layer comprising non-fibrous porous film on an upper surface of the adhesion layer; and coating a water-soluble polymer on an upper surface of the porous liquid-sample-developing layer.

2. The method of claim 1 wherein a non-fibrous porous film is laminated on an upper surface of the adhesion layer while an adhesion layer is being provided by coating a water-soluble polymer solution on an upper surface of the functional layer, thereby providing the porous liquid-sample-developing layer.

3. The method of claim 1 wherein an adhesion layer is provided by coating an adhesive agent on an upper surface of the functional layer, and then a non-fibrous porous film is laminated on an upper surface of the adhesion layer, thereby providing the porous liquid-sample-developing layer.

4. The method of any of claims 1 to 3 wherein the non-fibrous porous film is 6, 6-nylon; 6-nylon; acrylate copolymer; polyacrylate; polyacrylonitrile; polyacrylonitrile copolymer; polyamide, polyimide; polyamide-imide; polyurethane; polyether sulfone; polysulfone; a mixture of polyether sulfone and polysulfone; cellulose acylate; a saponified substance of cellulose acylate; polyester; polyester carbonate; polyethylene; polyethylene chlorotrifluoroethylene copolymer; polyethylene tetrafluoroethylene copolymer; polyvinyl chloride; polyolefin; polycarbonate; polytetrafluoroethylene; polyvinylidene difluoride; polyphenylene sulfide; polyphenylene oxide; polyfluorocarbonate; polypropylene; polybenzoimidazole; polymethyl methacrylate; styrene-acrylonitrile copolymer; styrene-butadiene copolymer; a saponified substance of ethylene-vinyl acetate copolymer; polyvinyl alcohol; or a mixture thereof.

5. The method of any of claims 1 to 4 wherein the non-fibrous porous film comprises polysulfone, polyether sulfone, cellulose acylate, 6,6-nylon, or 6-nylon.

6. The method of any of claims 1 to 5 wherein the non-fibrous porous film is an asymmetric film.

7. A dry multilayer analytical element for the analysis of a liquid sample, which is produced by the method of any of claims 1 to 6, and which comprises a water-non-transmitting planar support on one side of which at least one functional layer, an adhesion layer, at least one porous liquid-sample-developing layer comprising non-fibrous porous film, and a water-soluble polymer layer are integrally layered in the mentioned order.

## Patentansprüche

1. Verfahren zur Herstellung eines mehrschichtigen Trockenanalyseelements für die Analyse einer flüssigen Probe, das Bereitstellen wenigstens einer funktionellen Schicht an einer Oberfläche eines Wasser-nicht-übertragenden planaren Trägers; Bereitstellen einer Adhäsionsschicht an einer Oberseite der funktionellen Schicht; Bereitstellen wenigstens einer porösen flüssige Probe-entwickelnden Schicht, die einen nicht-faserigen porösen Film an einer Oberseite der Adhäsionsschicht umfasst, und Auftragen eines wasserlöslichen Polymers auf eine Oberseite der porösen flüssige Probe-entwickelnden Schicht umfasst.

2. Verfahren gemäß Anspruch 1, wobei ein nicht-faseriger poröser Film auf eine Oberseite der Adhäsionsschicht laminiert wird, während eine Adhäsionsschicht durch Auftragen einer wasserlöslichen Polymerlösung auf eine Oberseite der funktionellen Schicht angeordnet wird, wodurch die poröse flüssige Probe-entwickelnde Schicht bereitgestellt wird.

3. Verfahren gemäß Anspruch 1, wobei eine Adhäsionsschicht durch Auftragen eines Klebemittels auf eine Oberseite der funktionellen Schicht bereitgestellt wird und dann ein nicht-faseriger poröser Film auf eine Oberseite der Adhäsionsschicht laminiert wird, wodurch die poröse flüssige Probe-entwickelnde Schicht bereitgestellt wird.

4. Verfahren gemäß einem der Ansprüche 1 bis 3, wobei der nicht-faserige poröse Film 6,6-Nylon; 6-Nylon; Acrylatcopolymer; Polyacrylat; Polyacrylonitril; Polyacrylonitrilcopolymer; Polyamid, Polyimid; Polyamid-Imid; Polyurethan; Polyethersulfon; Polysulfon; ein Gemisch aus Polyethersulfon und Polysulfon; Celluloseacylat; eine verseifte Substanz von Celluloseacylat; Polyester; Polyestercarbonat; Polyethylen; Polyethylen-Chlortrifluorethylen-Copolymer; Polyethylen-Tetrafluorethylen-Copolymer; Polyvinylchlorid; Polyolefin; Polycarbonat; Polytetrafluorethylen; Polyvinylidendifluorid; Polyphenylensulfid; Polyphenylenoxid; Polyfluorcarbonat; Polypropylen; Polybenzimidazol; Polymethylmethacrylat; Styrol-Acrylonitril-Copolymer; Styrol-Butadien-Copolymer; eine verseifte Substanz von Ethylen-Vinylacetat-Copolymer; Polyvinylalkohol oder ein Gemisch davon ist.

5. Verfahren gemäß einem der Ansprüche 1 bis 4, wobei der nicht-faserige poröse Film Polysulfon, Polyethersulfon, Celluloseacylat, 6,6-Nylon oder Nylon-6 umfasst.

6. Verfahren gemäß einem der Ansprüche 1 bis 5, wobei der nicht-faserige poröse Film ein asymmetrischer Film ist.

7. Mehrschichtiges Trockenanalyseelement für die Analyse einer flüssigen Probe, das durch das Verfahren gemäß einem der Ansprüche 1 bis 6 hergestellt wird und das einen Wasser-nicht-übertragenden planaren Träger umfasst, auf dessen einer Seite wenigstens eine funktionelle Schicht, eine Adhäsionsschicht, wenigstens eine poröse flüssige Probe-entwickelnde Schicht, die einen nicht-faserigen porösen Film umfasst, und eine wasserlösliche Polymerschicht in der genannten Reihenfolge integral geschichtet sind.

## Revendications

1. Procédé pour produire un élément analytique multicouche sec pour l'analyse d'un échantillon liquide qui comprend la fourniture d'au moins une couche fonctionnelle sur une surface d'un support plan ne transmettant pas l'eau ; la fourniture d'une couche d'adhésion sur une surface supérieure de la couche fonctionnelle ; la fourniture d'au moins une couche poreuse de développement d'échantillon liquide comprenant un film poreux non fibreux sur une surface supérieure de la couche d'adhésion ; et l'application en revêtement d'un polymère soluble dans l'eau sur une surface supérieure de la couche poreuse de développement d'échantillon liquide.

2. Procédé selon la revendication 1 où un film poreux non fibreux est stratifié sur une surface supérieure de la couche d'adhésion tandis qu'une couche d'adhésion est fournie par application en revêtement d'une solution de polymère soluble dans l'eau sur une surface supérieure de la couche fonctionnelle, pour fournir la couche poreuse de développement d'échantillon liquide.

3. Procédé selon la revendication 1 où une couche d'adhésion est fournie par application en revêtement d'un agent adhésif sur une surface supérieure de la couche fonctionnelle, après quoi un film poreux non fibreux est stratifié sur une surface supérieure de la couche d'adhésion, pour fournir la couche poreuse de développement d'échantillon liquide.

4. Procédé selon l'une quelconque des revendications 1 à 3 où le film poreux non fibreux est le nylon 6,6 ; le nylon 6 ; un copolymère d'acrylate ; un polyacrylate ; un polyacrylonitrile ; un copolymère de polyacrylonitrile ; un polyamide ; un polyimide ; un polyamide-imide ; un polyuréthane ; une polyéthersulfone ; une polysulfone ; un mélange de polyéthersulfone et de polysulfone ; un acylate de cellulose ; une substance saponifiée d'acylate de cellulose ; un polyester ; un polyester carbonate ; un polyéthylène ; un copolymère polyéthylène chlorotrifluoroéthylène ; un copolymère polyéthylène tétrafluoroéthylène ; un poly(chlorure de vinyle) ; une polyoléfine ; un polycarbonate ; un polytétrafluoroéthylène ; un poly(difluorure de vinylidène) ; un poly(sulfure de phénylène) ; un poly(oxyde de phénylène) ; un polyfluorocarbonate ; un polypropylène ; un polybenzoimidazole ; un poly(méthacrylate de méthyle) ; un copolymère styrène-acrylonitrile ; un copolymère styrène-butadiène ; une substance saponifiée de copolymère éthylène-acétate de vinyle ; un poly(alcool vinylique) ; ou un mélange de ceux-ci.

5. Procédé selon l'une quelconque des revendications 1 à 4 où le film poreux non fibreux comprend une polysulfone, une polyéthersulfone, un acylate de cellulose, le nylon 6,6 ou le nylon 6.

6. Procédé selon l'une quelconque des revendications 1 à 5 où le film poreux non fibreux est un film asymétrique.

7. Elément analytique multicouche sec pour l'analyse d'un échantillon liquide, qui est produit par le procédé selon l'une quelconque des revendications 1 à 6, et qui comprend un support plan ne transmettant pas l'eau sur un côté duquel au moins une couche fonctionnelle, une couche d'adhésion, au moins une couche poreuse de développement d'échantillon liquide comprenant un film poreux non fibreux ; et une couche de polymère soluble dans l'eau sont disposées en couches d'une pièce dans l'ordre mentionné.
